# EUROPEAN PATENT APPLICATION

(11) **EP 4 750 091 A1**
(43) Date of publication of application: **27.05.2026**
(21) Application number: 25217721.7
(22) Date of filing: 21.11.2025
(51) Int. Cl.: H04R 25/00

(54) **METHOD FOR GENERATING HEARING AID EARMOLDS BASED ON ACOUSTIC AND PHYSICAL MODELS OF THE EAR CANAL**

(30) Priority: 21.11.2024 US 202463723384 P
(71) Applicant: Father Flanagan's Boys' Home doing Business as Boys Town National Research Hospital, Omaha, NE 68131 (US)
(72) Inventor: MCCREERY, Ryan, Nebraska, 68131 (US); MERCHANT, Gabrielle, Nebraska, 68131 (US); THELAGATHOTI, Rama, Nebraska, 68131 (US); SCOLLIE, Susan Diane, London, N6G 1H1 (CA); NIKAN, Soodeh, London, N6A 3K7 (CA); LADAK, Hanif, London, N6A 3K7 (CA); BEAULAC, Steve, London, N6G 1H1 (CA); HOLDEN, Matthew, London, N6G 1H1 (CA)
(74) Representative: Tomkinson, Alexandra

(57) **Abstract**

Acoustic models accurately predict ear-canal acoustics in children using standard clinical immittance measures to improve the accuracy of hearing aid fitting when measurements cannot be made in the child's ear canal. Physical models of ear canal geometry have been associated to indirect acoustic estimates, which makes is possible to develop models that can predict physical and acoustic characteristics of the ear canal. Such a model predicts ear-canal geometry and acoustics to product earmolds without the need for ear impressions or repeated verification. The combination of predicting ear canal geometry and acoustics improves the quality of hearing aid fitting in children, positively impacts developmental outcomes, and reduces the burden for children and their families. This is especially seen in rural areas, developing countries, and other areas where the ratio of patients to care provides is reduced.

## Description

### CROSS REFERENCE TO RELATED APPLICATIONS

This application claims priority under 35 U.S.C. § 119(e) to provisional patent application U.S. Serial No. 63/723,384, filed November 21, 2024. The provisional patent application is hereby incorporated by reference in its entirety herein, including without limitation: the specification, claims, and abstract, as well as any figures, tables, appendices, or drawings thereof.

### TECHNICAL FIELD

The present disclosure relates generally to systems, apparatus, and/or corresponding methods in at least the audiometry and hearing aid industries. More particularly, but not exclusively, the disclosure relates to systems, methods, and/or apparatus for generating hearing aid earmolds based on acoustic and physical models of ear canals.

### BACKGROUND

The background description provided herein gives context for the present disclosure. Work of the presently named inventors, as well as aspects of the description that may not otherwise qualify as prior art at the time of filing, are neither expressly nor impliedly admitted as prior art.

Over 3-million children in the U.S. have hearing loss in the mild-to-severe range (Wang et al. 2019; Feder et al. 2017) and over 80% of these children with hearing loss (CHL) are candidates for hearing aids (McCreery et al. 2020). Advances in newborn hearing screening, early diagnosis, and early intervention have reduced the median age of hearing aid fitting from 28-months (Harrison & Roush, 2003) to 7-months of age (Holte et al. 2012). Universal newborn hearing screening programs were established across the U.S. over two decades ago to minimize the developmental risks associated with hearing loss during critical developmental periods, including delays in spoken language (Tomblin et al. 2014; 2015; Walker et al 2019), cognition ( McCreery & Walker, 2022), and academic outcomes (Tomblin et al. 2020; 2021). Hearing aids are increasingly fitted within the first few months of life (Holte et al. 2012). One persistent challenge for maintaining consistent speech audibility in hearing aid fitting for infants and young children is that rapid ear-canal growth during the first few years of life (Keefe et al. 1994) requires frequent ear impressions and hearing aid verification to maintain optimal acoustic coupling between the child's ear canal and the hearing aid. New ear impressions currently require an in-person visit to an audiologist and a follow-up visit to replace the earmold and complete hearing aid verification. This process is recommended every 2-3 months in the first year of life and must be completed every 6 months until 3 years of age. Attendance at multiple audiology appointments is a significant challenge for many families of CHL (Petrarca & Worthington, 2022), and CHL whose families live a greater distance from specialized pediatric centers are at the greatest risk for limited follow-up (Spivak et al. 2009; Bush et al. 2014), likely leading to suboptimal fittings and outcomes. Furthermore, there are many places in the world where audiologists are not available to provide these aspects of the fitting process at all. This lack of follow-up after hearing aid fitting can lead to poorer speech audibility as the ear grows and the coupling with the child's earmold allows sound to leak out of the ear due to poor coupling.

Previous research has established the crucial role of well-fitted and consistently worn HAs to support *cumulative auditory experience* for CHL (McCreery et al. 2015; McCreery & Walker, 2022). CHL who have greater speech audibility and hours of hearing aid use have better language outcomes (Walker et al. 2015), academic achievement, (Tomblin et al. 2020), and speech recognition in noise (McCreery et al. 2019; Walker et al. 2019) than CHL with less audibility and device use.

Hearing aids are generally a highly effective and modifiable intervention for CHL. However, over 30% of CHL who were identified through newborn hearing screening and fitted with hearing aids experience persistent delays in speech recognition, language, and/or academic outcomes relative to peers with typical hearing, even in the absence of other developmental comorbidities (Tomblin et al. 2014; Tomblin et al. 2015; Wiseman et al. 2023). One major contributing factor to these persistent delays are related to inconsistent aided speech audibility through their hearing aids (McCreery et al. 2013; McCreery et al. 2015; Wiseman et al. 2024).

As noted, speech audibility provided by a hearing aid is dependent on maintaining a coupling between the hearing aid and the child's ear canal with an earmold. Current earmolds require a physical impression of the ear canal, often taken using a silicone material that is injected into the ear canal that hardens in a few minutes. Maintaining consistent aided audibility in young children requires frequent replacement of the earmolds that connect the hearing aid to the child's ear and electroacoustic verification to maintain the coupling needed to preserve sound transmission to the ear. Audibility with hearing aid must also be verified and optimized frequently using probe microphone measures of the child's hearing aid in the ear or of the child's wideband real-ear-to-coupler difference (wRECD; Bagatto et al. 2005; Vaisberg et al. 2018) to facilitate simulated real-ear measures in a coupler. Verification needs to occur every few months in infants and young children because of rapid changes in ear-canal growth (Keefe et al. 1994) that impact both earmold fit of the hearing aid to the ear canal and the acoustics of the ear canal, which impact sound transmission. Appointments to take impressions of ear canal and follow-up appointments to fit and verify the hearing aids occur multiple times per year. These frequent appointments are currently the clinical gold standard for the pediatric hearing aid fitting process but can create barriers to consistent hearing aid follow-up for families. In many parts of the developing world, CHL do not have access to audiologists or other trained professionals who can replace earmolds or complete verification at all (Olusanya et al. 2007).

Even in ideal healthcare systems, CHL often use hearing aids that are not well-fitted. Hearing aid output for CHL is prescribed using fitting formulae that normalize audibility (Scollie et al. 2005) based on a listener's pure-tone audiometric thresholds in each ear. Audiogram-based hearing aid prescriptions have been the standard of care for CHL for over five decades (Skinner, 1980; Cornelisse et al. 1995), but many children experience suboptimal hearing aid fitting outcomes that do not provide sufficient audibility to support spoken language development (McCreery et al. 2015; Wiseman et al. 2024). Over a third of CHL are at risk for language delays even when their hearing aids are consistently worn, and they have no developmental comorbidities beyond hearing loss (Wiseman et al. 2023). These persistent delays are even apparent in CHL with mild degrees of hearing loss (Walker et al. 2020).

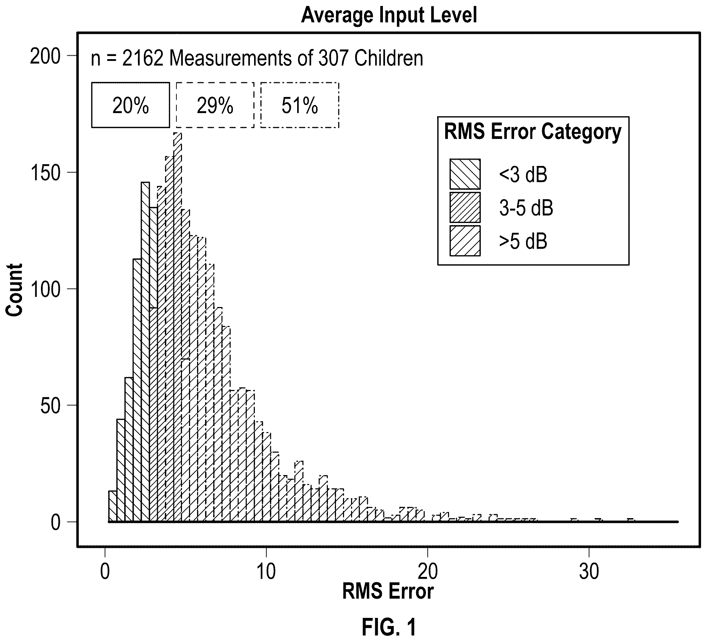

A major contributing factor to poorly fitted hearing aids in children is a lack of consistent earmold coupling as the child's ear grows rapidly during the first few years of life. Likewise, electroacoustic verification of audibility using probe microphone measures is essential to ensure consistent speech audibility through hearing aids over time. Probe microphone measures require an audiologist to place a small microphone in the child's ear canal to measure either the hearing aid output directly or the WRECD so that hearing aid verification can be completed in a coupler (Bagatto et al. 2005). However, these measurements can be challenging to obtain in young children. Even model pediatric hearing aid programs often can only complete hearing aid verification in 60% of cases for children who are below three years of age (Moodie et al. 2016), and ~40% of US audiologists who fitted children reported that they do not routinely measure hearing aid output or wRECD (McCreery et al. 2013). The result of these inconsistent practices is that more than 50% of CHL who were fitted with hearing aids in one recent study had suboptimal hearing aid fittings that compromised speech audibility (see, e.g., Fig. 1). Poorly fitted hearing aids are a major contributor to persistent delays in spoken language development in CHL (Tomblin et al. 2015; Wiseman et al. 2023). Thus, an alternative service delivery model that do not require frequent audiological follow-up and can maintain aided audibility over time as the child's ear canal grows would help to resolve this persistent problem.

Limited access to hearing aid verification is a major barrier to providing consistent audibility for CHL. Pediatric audiologists have specialized training in diagnostic assessment and intervention to provide care for CHL, but pediatric audiologists are often concentrated in urban or suburban areas at large hospitals or pediatric centers of excellence (Chung et al. 2017). Current clinical models for providing hearing aid require in-person visits for ear impressions followed by a separate clinical visit for hearing aid fitting and verification, despite the fact that telehealth is increasingly being used for screening and diagnostic stages of hearing care (Prins et al. 2021). Frequent visits for ear impressions and repeated hearing aid verification are a significant barrier for many CHL and their families to receive consistent follow-up care (Petrarca & Worthington, 2022). Families of CHL who live a greater distance from their care providers have less frequent access to care and may experience poorer outcomes as a result (Bush et al. 2014). Recent data shows that CHL who live further away from pediatric audiology services have fittings that deviate from prescriptive targets to a greater degree than CHL who live closer to pediatric audiology centers. Less frequent clinical visits mean that earmolds may not provide optimal acoustic coupling to the child's ear canal as they grow and that hearing aid output cannot be maintained at the same level over time. The situation is far more dire in developing countries. In Africa, for example, 18 of 42 countries do not have a single audiologist and the average number of audiologists across the continent is 1.4 audiologists per million people (WHO, 2024). Worldwide there are millions of CHL who cannot be adequately served by current hearing aid service delivery models and have poorer outcomes than if there were alternative models that could be implemented without frequent in-person interactions with pediatric audiologists.

Acoustic and physical models of the ear canal can be leveraged to fabricate earmolds and hearing aid verification without clinical visits. No other part of the auditory system has been more widely characterized than the ear canal. The physical and acoustic characteristics of the ear canal have been well-documented because of the importance for audiological assessment (Keefe et al, 1994; Voss & Hermann, 2005) and hearing aid fitting (Bagatto et al. 2002). The acoustic and physical attributes of the ear canal are highly correlated and have been validated across the lifespan using a wide range of direct measurement techniques including direct measurement (Voss et al. 2020), physical models (Stinson, 1985), and computerized tomography (Balouch et al. 2023). These modelling approaches have been applied descriptively or have been the basis of clinical procedures to measure wRECD. Existing modelling approaches have not been used to fabricate earmolds without an ear impression, forecast earmold growth, or to predict the acoustic characteristics of hearing aid fittings to estimate HA verification over time as child's ears grow. A recent patent application, U.S. Serial No. 18/449,844, hereby incorporated by reference in its entirety, discloses relatively simple models of predicting individual wRECD values from wideband immittance measures in a large group of infants and young children (McCreery et al. 2023a; McCreery et al. 2023b). These estimates produced predictions of ear-canal acoustics that were within 3 dB of a child's measured wRECD in nearly 90% of ears, including for children with abnormal middle ear function. However, it is not known whether these acoustic predictions could be combined with physical models of ear-canal geometry to produce earmolds for children that do not require ear impressions or frequent clinical visits for hearing aid verification.

Therefore, there is a need in the art for systems and methods of updating hearing aid earmolds quickly and even remotely.

### SUMMARY

The following objects, features, advantages, aspects, and/or embodiments are not exhaustive and do not limit the overall disclosure. No single embodiment need provide each and every object, feature, or advantage. Any of the objects, features, advantages, aspects, and/or embodiments disclosed herein can be integrated with one another, either in full or in part.

It is a primary object, feature, and/or advantage of the present disclosure to improve on or overcome the deficiencies in the art.

It is a further object, feature, and/or advantage of the present disclosure to train models that can be used to fabricate and forecast earmolds for hearing aid components based upon an existing earmold and/or one or more acoustic measurements. For example, immittance measurements (e.g., wideband acoustic immittance, WAI) and/or wRECD can be used as the acoustic measurements that are input to a machine-learned model that will predict the size and shape for hearing aid earmolds. The model can also define any needed change to hearing aid programming based on modeled/expected ear canal growth.

It is still yet a further object, feature, and/or advantage of the present disclosure to reduce the need for trained audiologist to be able to update hearing aid components.

The systems and/or methods disclosed herein can be used in a wide variety of applications. For example, while the models are disclosed for hearing aid components, generally any device that is used with an ear canal can benefit from the present disclosure.

According to some aspects of the present disclosure, a method for generating hearing aid earmolds comprises obtaining an acoustic measurement from an ear canal; and using a machine-learned model, generating an earmold for a hearing aid for a user, wherein the machine learned model has been trained by physical scans of ear impressions and one or more acoustic measurements to identify ear canal geometries and to classify the geometries based upon the inputs.

According to at least some embodiments, the model generated impression from a person's opposite ear can be mirrored to create a suitable earmold for their opposite ear.

According to at least some embodiments, the acoustic measurement comprises one of wideband acoustic immittance and wideband real-ear-to-coupler difference.

According to at least some embodiments, the acoustic measurement comprises both wideband acoustic immittance and wideband real-ear-to-coupler difference.

According to at least some embodiments, the step of generating the earmold comprises creating a physical earmold model.

According to at least some embodiments, the physical earmold model is created by 3D printing.

According to at least some embodiments, the method further comprises assessing the physical and acoustic effectiveness of the generated earmold.

According to at least some embodiments, the step of generating an earmold with the machine-learned model comprises predicting physical and acoustic characteristics of an ear canal of a user at one point in time or forecasting future ear-canal growth for increasing the size of the earmold or predicting the acoustic and physical characteristics of the opposite ear. Audiologists may have access to an impression of one ear, and the left and right ears of individual people are sufficiently similar to allow prediction of one ear from another.

According to at least some embodiments, the hearing aid earmold comprises an unvented, full-shell silicone, vinyl or other biocompatible material earmold for a behind-the-ear hearing aid.

According to additional aspects of the disclosure, a system for generating hearing aid molds comprises an apparatus for acquiring an acoustic measurement; a machined-learned model that has been trained by physical scans of ear impressions and one or more acoustic measurements to identify ear canal geometries and to classify the geometries based upon the inputs; wherein the acoustic measurement is used by the machine-learned model to generate a hearing aid mold based upon said acoustic measurement.

According to at least some embodiments, the acoustic measurement comprises one of wideband acoustic immittance and wideband real-ear-to-coupler difference.

According to at least some embodiments, the acoustic measurement comprises both wideband acoustic immittance and wideband real-ear-to-coupler difference.

According to at least some embodiments, the hearing aid mold comprises an unvented, full-shell silicone earmold for a behind-the-ear hearing aid.

According to at least some embodiments, the apparatus for acquiring an acoustic measurement comprises a clinical device.

According to at least some embodiments, the clinical device comprises a probe for interacting with an ear canal.

According to at least some embodiments, the system further comprises an additive manufacturing device that creates the generated hearing aid mold.

According to still additional aspects of the disclosure, a system for generating a hearing aid mold comprises a device for acquiring an acoustic measurement from an ear canal; the device connected to computer readable medium configured to: receive the acoustic measurement; compare the acoustic measurement with a machine-learned model that has been trained to identify classifiers, the classifiers being ear canal geometries associated with acoustic measurements and physical measurements; and generate the hearing aid mold for a user based upon the identified classifiers.

According to at least some embodiments, the acoustic measurement comprises one of wideband acoustic immittance and wideband real-ear-to-coupler difference.

According to at least some embodiments, the acoustic measurement comprises both wideband acoustic immittance and wideband real-ear-to-coupler difference.

According to at least some embodiments, the system further comprises an additive manufacturing device that creates the generated hearing aid mold.

According to at least some embodiments, the clinical device comprises a probe for interacting with an ear canal.

These and/or other objects, features, advantages, aspects, and/or embodiments will become apparent to those skilled in the art after reviewing the following brief and detailed descriptions of the drawings. The present disclosure encompasses (a) combinations of disclosed aspects and/or embodiments and/or (b) reasonable modifications not shown or described.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

Several embodiments in which the present disclosure can be practiced are illustrated and described in detail, wherein like reference characters represent like components throughout the several views. The drawings are presented for exemplary purposes and may not be to scale unless otherwise indicated.
Figure 1 is a graph showing the distribution of hearing aid fitting root-mean-square error (RMSe) for 307 CHL. Red bars (51% of cases) had hearing fittings that were > 5 dB RMSe from DSL prescription, yellow bars (29% of cases) had fittings with RMSe between 3-5 dB, and green bars (20%) of cases had fittings that optimized audibility with < 3 dB from prescription.
Figure 2 is a depiction of a digital scanning instrument used to create 3D scan of ear impressions.
Figure 3 is a schematic of a cloud-based computing infrastructure that can be used with aspects of the present disclosure for modeling and support of earmold configuration.

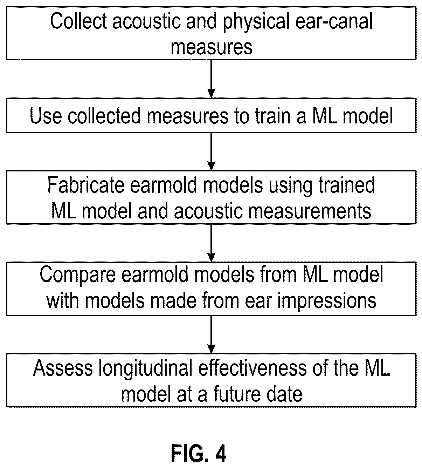

Figure 4 is a diagram showing steps of a process for developing and modeling hearing aids for children based on aspects of the present disclosure.

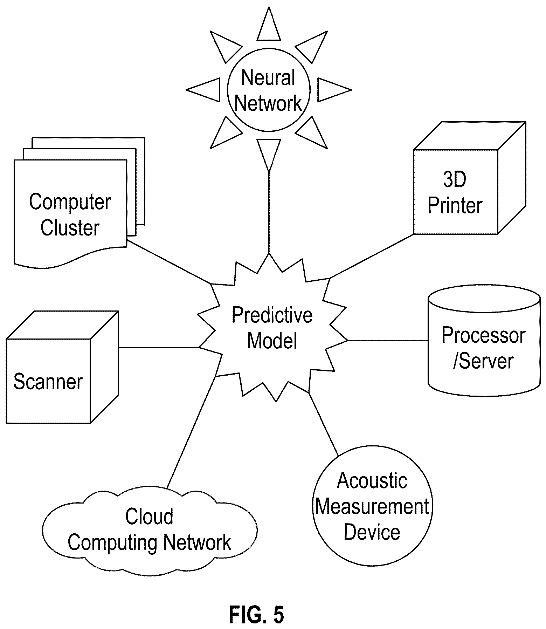

Figure 5 is a diagram showing components of a system for generating earmolds from a machine-learned model according to aspects of the present disclosure.

An artisan of ordinary skill in the art need not view, within isolated figure(s), the near infinite distinct combinations of features described in the following detailed description to facilitate an understanding of the present disclosure.

### DETAILED DESCRIPTION

Unless defined otherwise, all technical and scientific terms used above have the same meaning as commonly understood by one of ordinary skill in the art to which embodiments of the present disclosure pertain.

The terms "a," "an," and "the" include both singular and plural referents.

The term "or" is synonymous with "and/or" and means any one member or combination of members of a particular list.

As used herein, the term "exemplary" refers to an example, an instance, or an illustration, and does not indicate a most preferred embodiment unless otherwise stated.

The term "about" as used herein refers to slight variations in numerical quantities with respect to any quantifiable variable. Inadvertent error can occur, for example, through use of typical measuring techniques or equipment or from differences in the manufacture, source, or purity of components.

The term "substantially" refers to a great or significant extent. "Substantially" can thus refer to a plurality, majority, and/or a supermajority of said quantifiable variables, given proper context.

The term "generally" encompasses both "about" and "substantially."

The term "configured" describes structure capable of performing a task or adopting a particular configuration. The term "configured" can be used interchangeably with other similar phrases, such as constructed, arranged, adapted, manufactured, and the like.

Terms characterizing sequential order, a position, and/or an orientation are not limiting and are only referenced according to the views presented.

The term "real-ear-to-coupler difference" (RECD) means the occluded response of the ear canal. It is a measurement that compares the sound pressure level (SPL) in the ear canal to the SPL in a coupler that simulates the average adult ear canal.

The term "wideband acoustic immittance" (WAI) refers to a group of acoustic measurements that provide detailed mechanical and acoustic information (i.e., absorbance, power reflectance, admittance, and impedance, among others). WAI measurements are made in the ear canal in response to wideband stimuli (e.g., a click or chirp) and compare a sound input to the absorbed or reflected portions of that sound.

The term "wideband real-ear-to-coupler difference" (wRECD) is an instantiation of the RECD that can characterize ear canal acoustics at frequencies above 6000 Hz.

The term "static admittance" refers to the maximum compliance (mobility) of the middle ear system (i.e., the greatest amount of acoustic energy absorbed by the middle ear system (the vertical peak of the tympanogram tracing).

The term "absorbance" refers to a measure of the effectiveness of the middle ear (to absorb sound) as a function of frequency.

The term "hearing aid" or "hearing aid device" refers to a device designed to improve hearing by making sound audible to a person with hearing loss. Such hearing aid includes, but is not limited to, body-worn devices, behind the ear devices, in the ear devices, invisible-in-canal hearing aids, extended wear hearing aids, CROS hearing aids, eyeglass aids, osseointegrated auditory prosthesis (formerly called the bone-anchored hearing aid), cochlear implants. stethoscopes, and hearing aid applications.

The "scope" of the present disclosure is defined by the appended claims, along with the full scope of equivalents to which such claims are entitled. The scope of the disclosure is further qualified as including any possible modification to any of the aspects and/or embodiments disclosed herein which would result in other embodiments, combinations, subcombinations, or the like that would be obvious to those skilled in the art.

The present disclosure is not to be limited to that described herein. Mechanical, electrical, chemical, procedural, and/or other changes can be made without departing from the spirit and scope of the present disclosure. No features shown or described are essential to permit basic operation of the present disclosure unless otherwise indicated.

As will be understood, aspects and/or embodiments of the present disclosure include the measurements of ear-canal acoustics (wRECD and WAI) and ear impressions from children. The acoustic and physical measurements are used to create a predictive model using a deep neural network (DNN) that can produce earmolds and estimate HA verification across a wide age range of children. to solve a fundamental problem in provision of HAs to children.

As will be understood herein, aspects and/or embodiments of the present disclosure will allow acoustic measurements to be used to make ear molds for hearing aids. For example, at least some advantages of the disclosure will be to measure ear-canal acoustics (wRECD and wideband acoustic immittance) and ear impressions from children. The acoustic and physical measurements will be used to create a predictive model using a deep neural network (DNN) that can produce earmolds and estimate hearing aid (HA) verification across a wide age range of children to solve a fundamental problem in provision of HAs to children.

As will be understood, the present disclosure presents a major paradigm shift in prescribing and assessing HAs for CHL that will remove barriers from the intervention process for CHL who use hearing aids. The need to take frequent ear impressions during infancy and early childhood is a major challenge for many families of CHL to access consistent care that directly contributes to HA fittings that do not optimize audibility to support spoken language development. Generating earmolds from model-based estimates of ear-canal geometry and predicting ear-canal acoustics for HA verification without the need to take ear impressions or conduct on-site appointments will fundamentally change clinical HA provision for infants and young children. The approaches disclosed herein could be used to prospectively generate new earmolds and predict changes in HA output as the child grows over time.

To generate a machine-learned model to generate the earmolds, participants will be utilized to train the model. This will include children aged 3-months to 18-years old and include both children with hearing loss (CHL) and children with no substantial hearing loss (CNL). The broad range provides changes in ear-canal acoustics over a large age range. Both CNH and CHL will be used CHL because the acoustic and physical models are primarily based on acoustic and physical measures of the ear canal. Thus, this allows for a larger sample size.

Sample sizes for each process herein is based on simulation of clinical tolerances for acoustic (+/- 3 dB) and volumetric (+/- 3 mm³) characteristics of earmolds. The simulation is based on a linear mixed effects model framework that uses age-based variance parameters and an auto-regressive covariance structure across age where the correlation structure over time is high for ages that are close in time and decreases for more distant ages. Age in months is included in the simulation model. Effect sizes for wRECD and variance are derived from work by Bagatto et al. (2002) and McCreery et al. (2015; 2023). Effect sizes for physical ear-canal growth and size are estimated based on data from Voss et al. (2020) and Keefe et al. (1994). The data structure is simulated 1000 times and the sample size is estimated as the minimum value where the variance of wRECD and ear-canal size are co-predicted with minimal error across the age range.

Each child's wRECD is measured in both ears using an Audioscan Verifit 2 probe microphone system (or other like clinical device) and an insert foam ear tip. The wRECD is used as an input to the machine learning model to characterize ear-canal acoustics from 100 - 10,000 Hz in one-third octave bands.

WAI is measured in both ears for each child from 200 - 8,000 Hz in one-third octave bands. The WAI is used as an acoustic correlate of the wRECD as in U.S. Serial No. 18/449,844, which is hereby incorporated by reference in its entirety. The WAI is used to estimate the middle ear status of the child at the time of the measurement (normal, abnormal with an intact tympanic membrane, and abnormal with a tympanostomy tube or perforation).

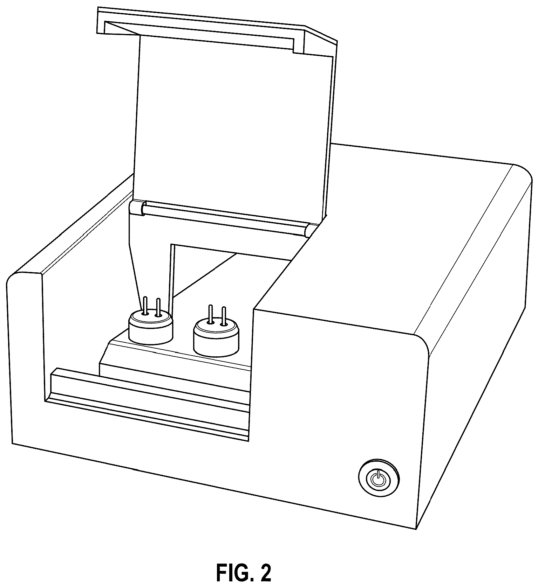

A licensed pediatric audiologist can be used to take impressions of both ears for each child using silicone impression material. The impressions for each ear will be digitally scanned using a Smart Optics Duo Scan (see, e.g., Fig. 2). However, it should be appreciated that other scanning apparatus capable of taking the required scans can be used. The Duo Scan uses white light LED to create high-resolution scans of ear impressions (accuracy < 9µm per ISO 12836) for both ears simultaneously in approximately 13-seconds. The digitized 3D scans are used as inputs to the machine-learning model for the physical aspects of the model. Ear scans are also used in the validation phase to assess model-generated earmolds.

Additional components that may be utilized for any of the embodiments and/or aspects of the disclosure include facilities with a double-walled, sound-treated booth, clinical audiometers, immittance devices, otoacoustic-emission systems, visual-reinforcement systems, and a variety of transducers, including supra-aural and insert earphones, bone vibrators, and loudspeakers. Equipment can include hearing-aid analyzers, otoscopes, and multiple PCs. The audiology testing area may be adjacent to a large individual testing room for completion of speech-language, cognitive, and vision measures. This testing suite can be equipped with a mounted SONY HD color digital video camera (EVI-H100V) with integrated pan head that can be remotely controlled and allows for observation and recording. The recording system can include a receiver, a Gefen MP4 recorder, and a DVD recorder. A Telex wireless lavaliere microphone with receivers and transmitters are used for audio recordings. This area has two IBM laptop computers with speakers for presentation of computer-automated tasks. Two separate stations are used for data analysis and include SALT (Systematic Analysis of Language Transcripts) and CLAN (Child Language Analysis) language transcript software.

The modeling approach utilized herein leverages both deep neural networks and ensemble learning techniques to handle the unique challenges of predicting ear canal growth. Since 3D scans of ear impressions are unstructured, nonlinear, and high-dimensional, aspects of the disclosure employ deep neural networks, specifically autoencoders and convolutional neural networks (CNNs), to extract and identify critical features within these complex datasets. For the structured ear-canal geometry measurements, ensemble learning methods such as random forests and support vector machines (SVMs) are applied to capture relevant growth patterns effectively. To mitigate the risk of overfitting, especially given the variability in the dataset, the disclosure can implement stratified k-fold cross-validation throughout model training. This validation approach will ensure that each fold represents the diversity of the data, enhancing model robustness. The final model, built as an ensemble of these approaches, is rigorously trained and validated to assess its accuracy and reliability for predicting ear canal dimensions, supporting its potential application in non-invasive hearing aid fittings for infants.

In addition, it should be appreciated that aspects and/or embodiments disclosed herein will utilize processors, memory, instructions, and the like, and will include a machine learning model or models to identify classifiers of aspects of ear conditions and/or pathologies. Machine learning (ML) is the study of computer algorithms that can improve automatically through experience and by the use of data. It is seen as a part of artificial intelligence. Machine learning algorithms build a model based on sample data, known as "training data", in order to make predictions or decisions without being explicitly programmed to do so.

While it is envisioned that generally any type of ML (e.g., supervised learning, unsupervised learning, semi-supervised learning, or reinforcement learning) can be utilized by any of the aspects and/or embodiments of the present disclosure utilize supervised learning. Supervised learning (SL) is the machine learning task of learning a function that maps an input to an output based on example input-output pairs. It infers a function from labeled training data consisting of a set of training examples. In supervised learning, each example is a pair consisting of an input object (typically a vector) and a desired output value (also called the supervisory signal). A supervised learning algorithm analyzes the training data and produces an inferred function, which can be used for mapping new examples. An optimal scenario will allow for the algorithm to correctly determine the class labels for unseen instances. This requires the learning algorithm to generalize from the training data to unseen situations in a "reasonable" way (see inductive bias). This statistical quality of an algorithm is measured through the so-called generalization error.

To solve a given problem of supervised learning, one has to perform the following steps: (1) Determine the type of training examples. Before doing anything else, the user should decide what kind of data is to be used as a training set. (2) Gather a training set. The training set needs to be representative of the real-world use of the function. Thus, a set of input objects is gathered, and corresponding outputs are also gathered, either from human experts or from measurements. (3) Determine the input feature representation of the learned function. The accuracy of the learned function depends strongly on how the input object is represented. Typically, the input object is transformed into a feature vector, which contains a number of features that are descriptive of the object. The number of features should not be too large, because of the curse of dimensionality; but should contain enough information to accurately predict the output. (4) Determine the structure of the learned function and corresponding learning algorithm. For example, the engineer may choose to use support-vector machines, regression analysis, or decision trees. (5) Complete the design. Run the learning algorithm on the gathered training set. Some supervised learning algorithms require the user to determine certain control parameters. These parameters may be adjusted by optimizing performance on a subset (called a validation set) of the training set, or via cross-validation. (6) Evaluate the accuracy of the learned function. After parameter adjustment and learning, the performance of the resulting function should be measured on a test set that is separate from the training set.

As will be understood, while generally any type of SL can be utilized, the example provided herein utilized three different classification algorithms to train the model, namely the support vector machine (SVM), k-Nearest Neighbors (k-NN), and classification ensemble (ENS).

Support-vector machines (SVMs, also support-vector networks) are supervised learning models with associated learning algorithms that analyze data for classification and regression analysis. Given a set of training examples, each marked as belonging to one of two categories, an SVM training algorithm builds a model that assigns new examples to one category or the other, making it a non-probabilistic binary linear classifier (although methods such as Platt scaling exist to use SVM in a probabilistic classification setting). SVM maps training examples to points in space so as to maximize the width of the gap between the two categories. New examples are then mapped into that same space and predicted to belong to a category based on which side of the gap they fall.

The k-nearest neighbors algorithm (k-NN) is a non-parametric classification method. k-NN is a type of classification where the function is only approximated locally, and all computation is deferred until function evaluation. Since this algorithm relies on distance for classification, if the features represent different physical units or come in vastly different scales then normalizing the training data can improve its accuracy dramatically.

Classification ensemble may also be referred to as ensemble learning. Ensemble methods use multiple learning algorithms to obtain better predictive performance than could be obtained from any of the constituent learning algorithms alone. Unlike a statistical ensemble in statistical mechanics, which is usually infinite, a machine learning ensemble consists of only a concrete finite set of alternative models, but typically allows for much more flexible structure to exist among those alternatives.

The trained model and the associated machine learning and application of the model will utilize processors, modules, memories, databases, networks, and potentially user interfaces to show the results and allow changes to be made.

In communications and computing, a computer readable medium is a medium capable of storing data in a format readable by a mechanical device. The term "non-transitory" is used herein to refer to computer readable media ("CRM") that store data for short periods or in the presence of power such as a memory device. It is envisioned that the clinical tools used for collecting the acoustic measurements and/or associated processors for evaluating the data from the clinical tools could implement CRM.

One or more embodiments described herein can be implemented using programmatic modules, engines, or components. A programmatic module, engine, or component can include a program, a sub-routine, a portion of a program, or a software component or a hardware component capable of performing one or more stated tasks or functions. A module or component can exist on a hardware component independently of other modules or components. Alternatively, a module or component can be a shared element or process of other modules, programs, or machines.

The system will preferably include an intelligent control (i.e., a controller) and components for establishing communications. Examples of such a controller may be processing units alone or other subcomponents of computing devices. The controller can also include other components and can be implemented partially or entirely on a semiconductor (e.g., a field-programmable gate array ("FPGA")) chip, such as a chip developed through a register transfer level ("RTL") design process.

A processing unit, also called a processor, is an electronic circuit which performs operations on some external data source, usually memory or some other data stream. Nonlimiting examples of processors include a microprocessor, a microcontroller, an arithmetic logic unit ("ALU"), and most notably, a central processing unit ("CPU"). A CPU, also called a central processor or main processor, is the electronic circuitry within a computer that carries out the instructions of a computer program by performing the basic arithmetic, logic, controlling, and input/output ("I/O") operations specified by the instructions. Processing units are common in tablets, telephones, handheld devices, laptops, user displays, smart devices (TV, speaker, watch, etc.), and other computing devices.

As noted, the clinical tool itself could include a processor, and/or the tool could be connected, either wired or wirelessly to a separate system encompassing the processing unit.

The memory includes, in some embodiments, a program storage area and/or data storage area. The memory can comprise read-only memory ("ROM", an example of nonvolatile memory, meaning it does not lose data when it is not connected to a power source) or random access memory ("RAM", an example of volatile memory, meaning it will lose its data when not connected to a power source). Examples of volatile memory include static RAM ("SRAM"), dynamic RAM ("DRAM"), synchronous DRAM ("SDRAM"), etc. Examples of nonvolatile memory include electrically erasable programmable read only memory ("EEPROM"), flash memory, hard disks, SD cards, etc. In some embodiments, the processing unit, such as a processor, a microprocessor, or a microcontroller, is connected to the memory and executes software instructions that are capable of being stored in a RAM of the memory (e.g., during execution), a ROM of the memory (e.g., on a generally permanent basis), or another non-transitory computer readable medium such as another memory or a disc.

In the instant case, the memory could include the machine learned classifiers and analog models, so as to fit the parameters of the model and to quickly and accurately identify the results based on the trained classifiers.

Generally, the non-transitory computer readable medium operates under control of an operating system stored in the memory. The non-transitory computer readable medium implements a compiler which allows a software application written in a programming language such as COBOL, C++, FORTRAN, or any other known programming language to be translated into code readable by the central processing unit. After completion, the central processing unit accesses and manipulates data stored in the memory of the non-transitory computer readable medium using the relationships and logic dictated by the software application and generated using the compiler.

In one embodiment, the software application and the compiler are tangibly embodied in the computer-readable medium. When the instructions are read and executed by the non-transitory computer readable medium, the non-transitory computer readable medium performs the steps necessary to implement and/or use the present invention. A software application, operating instructions, and/or firmware (semi-permanent software programmed into read-only memory) may also be tangibly embodied in the memory and/or data communication devices, thereby making the software application a product or article of manufacture according to the present invention.

The database is a structured set of data typically held in a computer. The database, as well as data and information contained therein, need not reside in a single physical or electronic location. For example, the database may reside, at least in part, on a local storage device, in an external hard drive, on a database server connected to a network, on a cloud-based storage system, in a distributed ledger (such as those commonly used with blockchain technology), or the like.

It is envisioned that the machine learned model and any of the training of the same could include cloud computing. Cloud computing is a model of service delivery for enabling convenient, on-demand network access to a shared pool of configurable computing resources (e.g., networks, network bandwidth, servers, processing, memory, storage, applications, virtual machines, and services) that can be rapidly provisioned and released with minimal management effort or interaction with a provider of the service.

A cloud computing environment is service oriented with a focus on statelessness, low coupling, modularity, and semantic interoperability. At the heart of cloud computing is an infrastructure comprising a network of interconnected nodes.

As noted, the training model could be implemented on a user interface. The interface could also be a point on introduction of data, such as training data or test data to compare to the trained model for analysis. The results of the comparison could then be shown on a user interface. The user interface could be the clinical device/tool itself, which can include a display or other interface to be used for the collection and review of the information obtained by the acoustic device.

A user interface is how the user interacts with a machine. The user interface can be a digital interface, a command-line interface, a graphical user interface ("GUI"), oral interface, virtual reality interface, or any other way a user can interact with a machine (user-machine interface). For example, the user interface ("UI") can include a combination of digital and analog input and/or output devices or any other type of UI input/output device required to achieve a desired level of control and monitoring for a device. Examples of input and/or output devices include computer mice, keyboards, touchscreens, knobs, dials, switches, buttons, speakers, microphones, LIDAR, RADAR, etc. Input(s) received from the UI can then be sent to a microcontroller to control operational aspects of a device.

The user interface module can include a display, which can act as an input and/or output device. More particularly, the display can be a liquid crystal display ("LCD"), a light-emitting diode ("LED") display, an organic LED ("OLED") display, an electroluminescent display ("ELD"), a surface-conduction electron emitter display ("SED"), a field-emission display ("FED"), a thin-film transistor ("TFT") LCD, a bistable cholesteric reflective display (i.e., e-paper), etc. The user interface can also be configured with a microcontroller to display conditions or data associated with the main device in real-time or substantially real-time.

Any components of the system could be connected via network or other communication protocol to transfer information, communicate with other systems, or provide other connectivity. In some embodiments, the network is, by way of example only, a wide area network ("WAN") such as a TCP/IP based network or a cellular network, a local area network ("LAN"), a neighborhood area network ("NAN"), a home area network ("HAN"), or a personal area network ("PAN") employing any of a variety of communication protocols, such as Wi-Fi, Bluetooth, ZigBee, near field communication ("NFC"), etc., although other types of networks are possible and are contemplated herein. The network typically allows communication between the communications module and the central location during moments of low-quality connections. Communications through the network can be protected using one or more encryption techniques, such as those techniques provided by the Advanced Encryption Standard (AES), which superseded the Data Encryption Standard (DES), the IEEE 802.1 standard for port-based network security, pre-shared key, Extensible Authentication Protocol ("EAP"), Wired Equivalent Privacy ("WEP"), Temporal Key Integrity Protocol ("TKIP"), Wi-Fi Protected Access ("WPA"), and the like.

For wired communications with a clinical device, it is envisioned that various protocols be used. For example, Ethernet could be used to transmit data from the clinical device to a remote location. Ethernet is a family of computer networking technologies commonly used in local area networks ("LAN"), metropolitan area networks ("MAN"), and wide area networks ("WAN"). Systems communicating over Ethernet divide a stream of data into shorter pieces called frames. Each frame contains source and destination addresses, and error-checking data so that damaged frames can be detected and discarded; most often, higher-layer protocols trigger retransmission of lost frames. As per the OSI model, Ethernet provides services up to and including the data link layer. Ethernet was first standardized under the Institute of Electrical and Electronics Engineers ("IEEE") 802.3 working group/collection of IEEE standards produced by the working group defining the physical layer and data link layer's media access control ("MAC") of wired Ethernet. Ethernet has since been refined to support higher bit rates, a greater number of nodes, and longer link distances, but retains much backward compatibility. Ethernet has industrial application and interworks well with Wi-Fi. The Internet Protocol ("IP") is commonly carried over Ethernet and so it is considered one of the key technologies that make up the Internet.

Still other types of wired communication, as well as removable memory devices, could be used. For example, USB, micro-USB, USB-C, lightning, and the like, are all ways to transmit data, and can be used with any of the aspects and/or embodiments provided.

Aspects of the disclosure will include the use of data processing and analytics, which can include high-performance workstations for processing imaging data and a 180+ terabyte array for data storage and resource sharing. Each workstation can be fully equipped with the latest software for advanced data analyses. A variety of specialized softwares can be used, including MATLAB, Analysis for Functional Neuroimages (AFNI), Statistical Parametric Mapping (SPM12), Brain Electrical Source Analysis (BESA), R, Statistical Package for the Social Sciences (SPSS), and others. Note that these are for illustrative purposes and is not limiting to the disclosure.

Additional equipment that can be utilized can include, a computing system comprised of four Dell PowerEdge nodes with 256 cores (Intel Xeon Turbo Platinum, 2.6 GHz, 11.2 GT/s), 2.0 TB of RAM (dual-rank, 3200 MT/s), high-speed GPUs (NVIDIA Ampere A100, 80 GB, Double Wide - Full Height), over 25 TB of high-speed data storage (Dell Enterprise, NVMe, U2, G4, P5600), and ultra-high speed networking capabilities (Broadcom 57414, dual-port, 10/25 GbE PCle). The GPUs are designed to maximize total throughput for machine learning, deep learning, and other artificial intelligence applications. The overall Orion cluster is implemented on a Rocky Linux platform with the Slurm workload manager, which helps allocate resources as jobs are set up and assigned to the cluster. However, it should be noted that generally any type of capable computer/computing system/processor be used with any of the aspects and/or embodiments of the present disclosure.

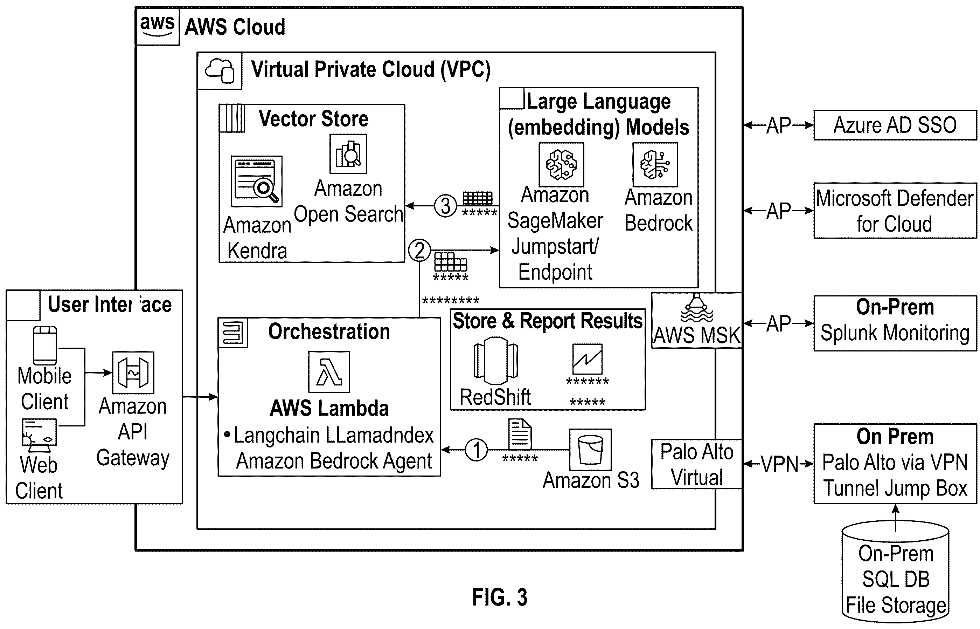

As shown in Fig. 3, an example of a cloud-based Al computing structure is provided. The system allows for rapid deployment of Al modelling and data analysis, which is provided by Amazon Web Services. The system can be used for scaling purposes but is not required in all embodiments.

The disclosure, as understood, includes the generation of earmolds for hearing aids. Such earmolds can be generated and fabricated using additive manufacturing, such as 3D printing. According to some embodiments, a Selective Laser Sintering (SLS) system can be used to fabricate the earmolds. The printer can print a wide array of materials, including nylon-based polymers, as well as carbon fiber polymer blends. Additional tools can be used to finalize completion of the fabricated earmolds as well. In addition, generally any type of additive manufacturing (i.e., 3D printing) can be used, including different types of materials for the earmolds. It should be noted that generally any type of biocompatible material could be used to 3D print the earmolds.

Therefore, as will be understood, the following methods, systems, etc. will provide for the training of a ML model that can then be used to generate earmolds based on acoustic measurements, thus eliminating the need for updated ear impressions, which will greatly increase the use of hearing aids and the effectiveness of the same. Currently, infants and young children who are fitted with hearing aids require multiple ear impressions and follow-up appointments for hearing aid verification as their ear canals grow. Current acoustic and physical models of the ear canal are robust but have not been leveraged to generate earmolds for hearing aids.

The following presents an example process and associated components that achieve at least some objectives of the disclosure.

A first step in a process according to the present disclosure will be to create a machine learning model based on acoustic measurements of the ear canal (wRECD and/or WAI) and physical measures based on 3D scans of silicon ear impressions. The main outcomes for this initial step are to train the machine learning model and cross validate the model predictions using both acoustic and physical estimates from the model against a new set of earmolds.

To predict ear-canal growth and related changes in acoustics, the process includes collecting wideband acoustic immittance, real-ear-to-coupler difference (RECD), measures of hearing aid output, and three-dimensional scans of ear impressions for a large cohort of CHL who use hearing aids. This data will be used to train a deep neural network (DNN) to predict earmold dimensions and the real-ear-to-coupler difference for a novel group of children with HL who use hearing aids.

For example, 500 CNH (1000 ears) provide adequate power to train the neural network models to predict acoustic and physical attributes of individual ears in child. The sampling approach includes to oversample children under 5-years of age (~300 CNH), given the significant variability in ear-canal growth during this age range and less variability over time and age than for children over 5-years of age (~200 CNH).

As described herein, wRECD is measured with an insert foam tip and WAI will be measured using the Interacoustics Titan immittance system (or like clinical apparatus). Physical scans of ear impressions are used to create a 3D model of each participant's ear canal. The acoustic and physical measurements for each ear of each participant are linked to generate the model. The participant age, sex, and middle ear status (normal, abnormal, or tube/perforation) is also used as inputs to the model.

Linear mixed effects models with random intercepts for each participant and ear are used to analyze the effects of age, sex, and middle ear status on acoustic (wRECD and/or WAI) and physical (physical volume and ear-canal cross-sectional area) characteristics of the ear canal. Random effects for repeated measures from the same participant allow inclusion of both ears with a model structure that reflects the high correlation between ears of the same participant. The neural network is trained on the acoustic and physical attributes of each ear. The process of k-fold cross validation provides an estimate for the performance of the model to predict both acoustic and physical ear-canal characteristics of unseen data from the model.

A next step is to fabricate and assess earmold acoustic and physical fit for children. Earmolds will be generated for a new group of children based on the model and compared to earmolds taken by an audiologist. The model-generated earmolds will be compared to the impression-generated earmolds using physical measurements of cross-sectional area and volume, as well as acoustic measurements of earmold fit using RECD. Audibility for hearing aid fitting by audiologists will be compared to model-predicted hearing aid fitting using the aided Speech Intelligibility Index (SII; ANSI S3.5-1997) and root-mean-square error (RMSe; McCreery et al. 2013) of the fittings based on Desired Sensation Level (DSL; Scollie et al. 2005) pediatric hearing aid prescription.

An additional aspect of the fabrication systems and methods as disclosed herein includes the ability for ear mirroring. "Earmold mirroring" refers to a method in 3D scanning and printing where the design of a single ear impression is digitally mirrored to create a mold for the opposite ear. Any of the systems or methods disclosed can be used to generate an appropriate earmold for a first ear (i.e., a left or right ear). Instead of having the same process be required to generate the other earmold, it is considered that the disclosure includes ear mirroring. Generally, left and right ears are common to one another in structure, but are mirror images of one another. Therefore, the present disclosure includes the ability to generate one earmold, such as for a left or right ear, and then to mirror the mold to fit the opposite ear. This will reduce the amount of time required and will aid in speeding up the process for creation of both ear molds for children or other users who utilize hearing aids for both ears. The mirroring can be done in a number of ways. Digital mirroring: Specialized software is used to create a mirror image of this 3D model. This process digitally flips the ear's shape so it's an exact mirror of the original. 3D printing: The mirrored 3D model is then used to 3D print a custom-fit earmold or shell for the opposite ear.

One goal of a machine learning model to predict physical and acoustic characteristics of the ear canal in children is to use the models to generate a predicted earmold and ear-canal acoustics that would limit the need for physical ear impressions and frequent hearing aid verification as the child's ear canal grows. At this step, the machine learning model developed herein will be used to generate an earmold for a new group of children and validate the acoustic and physical fit of the model-generated earmold using standard clinical metrics compared to an earmold generated from a commercial lab from the same ear impression.

A number of participants (e.g., 40 CNH or 80 ears) who do not participate in the model development process provide an adequate number to assess the acoustic and physical characteristics of model-generated earmolds compared to conventional earmolds.

As in the model generation process herein, an ear impression will be taken of each ear. The ear impression will be scanned using the Duoscan to create a 3D model of the ear canal. wRECD and WAI are also measured as before. The impressions can be sent to a commercial earmold manufacturer to fabricate an unvented, full-shell silicone earmold for a behind-the-ear hearing aid. The child's age, sex, and wRECD and WAI data for each ear is used as inputs to the machine learning model to generate a model-predicted ear impression. The model-predicted ear impression is used to 3D print an unvented, full-shell silicone earmold for a behind-the-ear hearing aid. The main outcome measures are the wRECD measured with the commercial and model-generated earmold. The model-generated and commercial earmolds can also undergo 3D scanning to assess the total volume and cross-sectional area of the ear canal for each earmold to assess the similarities in physical characteristics.

Linear mixed effects models can be used to evaluate the difference in wRECD between the model-generated earmold and the commercial earmold for each participant with random effects for each ear nested within participant. It is predicted that the model-generated earmold will produce a wRECD that is within 3 dB of the wRECD from the commercial earmold at one-third octave bands from 250 Hz - 10,000 Hz. 3 dB is chosen as the criterion as this has been the standard for test-retest reliability for RECD measures in previous studies (Bagatto et al. 2002). There are no clinical standards for physical estimates of the ear canal, so we rely on estimates from previous modelling studies of ear-canal geometry and ear-canal cross-sectional area (+/- 3 mm³). The physical and acoustic differences between model-generated and commercial earmolds will be assessed by age, participant sex, and middle ear status.

Still further, it should be considered that additional or alternative forecasting processes be used as part of the overall methods and/or systems of the disclosure. For example, aspects of the disclosure have been provided that utilize WAI or other acoustic measurements to be input into a model (such as ML or Al model) that is used to predict the earmold configuration. In addition, or in the alternative, the systems and methods of the present disclosure include the use of growth trajectories in the calculus to determine the size and shape of future iterations of earmolds for hearing aids. There are a number of growth charts that have been developed including but not limited to the Center of Disease Control and Prevention, World Health Organization, etc., that project a child's growth and that have been created based upon data accumulated over time. These charts can be used in any of the analyses to forecast growth of a child, which will be used in determining future earmold size and/or configuration. According to at least some aspects, the growth charts can be added to the ML and/or Al models as an additional form of training inputs for the models that can be used to aid in the output of the models. However, the models need not be used in all instances, and the growth charts could be used in place of as well as in addition to the models.

Additional considerations will include the range of silicone materials available for the 3D printers, which may limit the effectiveness of the earmolds. At some point, it is ideal to optimize or select one or more materials. However, it should be noted that any material may be used for the present disclosure. For example, as noted, generally any biocompatible material could be used in any of the manufacturing processes (e.g., 3D printing) in order to manufacture the ear molds.

Next, the process includes the assessment of the longitudinal effectiveness of the trained model. Once the model is tested and validated in real time or otherwise contemporaneous timeframes, the model can be used to determine changing ear canal structures. To accomplish this, a group of tested participants can be selected at a later time (e.g., 1-year) to determine the efficacy of the model to determine or predict changes to ear canal structure based on aging.

Such a process can include the following steps. Each of the participants in this step can undergo new ear impressions to allow fabrication of commercial earmolds for each ear. Model-generated earmolds will be generated from a 3D scan of the child's previous ear impression or a 3D scan of their earmold from at least one-year ago with their current age. As before, model-generated earmolds and commercial earmolds will be compared in terms of physical (volumetric measures from 3D scan) and acoustic (RECD) characteristics.

Linear mixed models with random intercepts for each participant and ear will be used to assess the acoustic and physical differences between model-generated earmolds based on previous ear impressions and commercial earmolds fabricated from a current ear impression. The modelling framework and criteria are the same as described before herein.

Longitudinal prediction of earmolds may be more variable than the concurrent/contemporaneous model-generated earmolds. If the model-generated earmold fit is not acceptable acoustically or physically, the process can incorporate current measures of RECD or WAI for the model input to see if using current ear-canal acoustics helps to improve model-predicted earmolds. These acoustic measures are less invasive than ear impressions and could be done in a community health worker context in places where pediatric audiologists are not available.

Therefore, systems and methods for using a machine-learned and/or Al model to predict earmolds for hearing aids have been shown and/or described. Such model can utilize one or more acoustic measurements to update the earmolds, which will promulgate a more accurate and updated hearing aid for users, which will correlate to better development for the user. In addition, as noted, the modeling can be used to forecast future ear-canal growth for increasing the size of the earmold.

It should be noted that any of the components of any of the aspects and/or embodiments provided can be combined with other components, even if not explicitly disclosed, to create yet additional embodiments that are within the scope of the present disclosure. In addition, variations and alternatives obvious to those skilled in the art are to be considered a part of the present disclosure.

## Claims

1. A method for generating hearing aid earmolds, comprising:
obtaining an acoustic measurement from an ear canal; and
using a machine-learned model and/or an Al model, generating an earmold for a hearing aid for a user, wherein the machine learned model has been trained by physical scans of ear impressions and one or more acoustic measurements to identify ear canal geometries and to classify the geometries based upon the inputs.

2. The method of claim 1, wherein the acoustic measurement comprises one of wideband acoustic immittance and wideband real-ear-to-coupler difference.

3. The method of claim 1, wherein the acoustic measurement comprises both wideband acoustic immittance and wideband real-ear-to-coupler difference.

4. The method of claim 1, wherein the step of generating the earmold comprises creating a physical earmold model.

5. The method of claim 4, wherein the physical earmold model is created by 3D printing.

6. The method of claim 1, further comprising after generating an ear mold for a first ear, predicting the ear mold for a second ear of the user, wherein the first ear is a left or right ear and the second ear is the opposite of the first ear of the user.

7. The method of claim 1, wherein the step of generating an earmold with the machine-learned model and/or Al model comprises predicting physical and acoustic characteristics of an ear canal of a user at one point in time or forecasting future ear-canal growth for increasing the size of the earmold.

8. The method of claim 1, wherein the hearing aid earmold comprises an unvented, full-shell silicone, vinyl, or other biocompatible material earmold for a behind-the-ear hearing aid.

9. A system for generating hearing aid molds, comprising:
an apparatus for acquiring an acoustic measurement;
a machined-learned or Al model that has been trained by physical scans of ear impressions and one or more acoustic measurements to identify ear canal geometries and to classify the geometries based upon the inputs;
wherein the acoustic measurement is used by the machine-learned model to generate a hearing aid mold based upon said acoustic measurement.

10. The system of claim 9, wherein the acoustic measurement comprises one of wideband acoustic immittance and wideband real-ear-to-coupler difference.

11. The system of claim 9, wherein the acoustic measurement comprises both wideband acoustic immittance and wideband real-ear-to-coupler difference.

12. The system of claim 9, wherein the hearing aid mold comprises an unvented, full-shell silicone earmold for a behind-the-ear hearing aid.

13. The system of claim 9, wherein the apparatus for acquiring an acoustic measurement comprises a clinical device.

14. The system of claim 13, wherein the clinical device comprises a probe for interacting with an ear canal.

15. The system of claim 9, further comprising an additive manufacturing device that creates the generated hearing aid mold.

16. A system for generating a hearing aid mold, comprising:
a device for acquiring an acoustic measurement from an ear canal;
the device connected to computer readable medium configured to:
receive the acoustic measurement;
compare the acoustic measurement with a machine-learned model that has been trained to identify classifiers, the classifiers being ear canal geometries associated with acoustic measurements and physical measurements; and
generate the hearing aid mold for a user based upon the identified classifiers.

17. The system of claim 16, wherein the acoustic measurement comprises one of wideband acoustic immittance and wideband real-ear-to-coupler difference.

18. The system of claim 16, wherein the acoustic measurement comprises both wideband acoustic immittance and wideband real-ear-to-coupler difference.

19. The system of claim 16, further comprising an additive manufacturing device that creates the generated hearing aid mold.

20. The system of claim 16, wherein the device further configured to forecast a future hearing aid mold of the user based upon growth trajectories of the user.
